# EUROPÄISCHE PATENTSCHRIFT

(11) **EP 0 365 970 B1**
(45) Veröffentlichungstag und Bekanntmachung des Hinweises auf die Patenterteilung: **04.08.1993**
(21) Anmeldenummer: 89119202.3
(22) Anmeldetag: 17.10.1989
(51) Int. Cl.: C07C 231/02, C07C 233/58

(54) **Verfahren zur Herstellung von Cyclopropancarbonsäureamid**
Process for the preparation of cyclopropanamide
Procédé pour la préparation de cyclopropanamide

(30) Priorität: 28.10.1988 DE 3836782
(43) Veröffentlichungstag der Anmeldung: 02.05.1990
(73) Patentinhaber: BAYER AG, 51368 Leverkusen (DE)
(72) Erfinder: Diehl, Herbert, Dr., D-5090 Leverkusen 1 (DE); Blank, Heinz Ulrich, Dr., D-5068 Odenthal-Glöbusch (DE); Ritzer, Edwin, Dr., D-4390 Gladbeck (DE)

(56) Entgegenhaltungen:
- EP-A- 0 205 403
- DE-A- 1 939 759
- GB-A- 1 304 667

## Beschreibung

Die vorliegende Erfindung betrifft ein Verfahren zur Herstellung von Cyclopropancarbonsäureamid durch Umsetzung von Cyclopropancarbonsäureestern mit NH₃ in Gegenwart von katalytischen Mengen eines Alkoholats, wobei unter Ausschluß eines Kohlenwasserstoff-Lösungsmittels gearbeitet wird.

Cyclopropancarbonsäureamid ist ein organisches Zwischenprodukt, welches, beispielsweise nach Umwandlung in das Cyclopropylamin, zu Wirkstoffen im Pharma- und Pflanzenschutzbereich umgesetzt wird. Ein solcher Einsatz fordert die Herstellung aller Zwischenprodukte in hohen Reinheiten, die bei bisherigen Herstellungsverfahren nicht erreicht wurden, so daß sich zusätzliche, ausbeutemindernde Reinigungsschritte anschließen mußten.

Die Umsetzung von Estern mit Ammoniak zu den zugehörigen Amiden ist im Prinzip bekannt; bei den bekannten Fällen wird stets ein Lösungsmittel oder ein Lösungsmittelgemisch verwendet.

So wird in DE-OS 19 39 759 (Äquivalent zu US 3.711.549) ein Gemisch von 0,15 Mol Natriummethylat und 0,95 Mol Cyclopropancarbonsäuremethylester in 40 ml Methanol und 390 ml Toluol mit Ammoniak bei etwa 80°C umgesetzt. Hierbei werden 85-90 % Umsatz erzielt, wobei dieses Ergebnis aber nur erreicht wird, wenn die bevorzugte Methanolkonzentration von 40-80 ml/Mol des Esters, der wiederum in einer 20-25 %igen Lösung in Toluol eingesetzt wird, eingehalten wird. Wenn das Toluol fortgelassen wird, erhält man niedrigere Amidausbeuten. Desgleichen sinkt der Umwandlungsgrad auf etwa 59 % ab, wenn man das Methanol fortläßt. Bei einer großtechnischen Ausführungsvariante erreicht der Arbeitsdruck 28 atm. Aus der unter diesen Bedingungen bestenfalls erzielbaren Umsetzungsrate von 90 % folgt die Notwendigkeit der destillativen Aufarbeitung, wobei Methanol, Toluol und nicht umgesetzter Cyclopropancarbonsäuremethylester wieder zurückgeführt werden müssen. Der bei einer solchen Destillation erhaltene Sumpfrückstand, der aus Cyclopropancarbonsäureamid, restlichem Toluol und Natriummethanolat besteht, wird sodann mit Wasser und Salzsäure versetzt, da das Amid im alkalischen, wäßrigen Milieu verseifungsanfällig ist; die wäßrige Phase wird im Scheidetrichter abgetrennt, und die organische Phase wird nochmals mit Wasser extrahiert. Das Cyclopropancarbonsäureamid befindet sich dann in der so erhaltenen, stark verdünnten wäßrigen Lösung.

In EP 205 403 wird die Umsetzung von sterisch behinderten sekundären und tertiären Cyclopropancarbonsäureestern mit Ammoniak unter Benutzung eines anderen Katalysators, nämlich des Natriumsalzes von mehrwertigen Alkoholen, wie Glykol oder Glycerin, beschrieben. Hierbei muß überschüssiger mehrwertiger Alkohol zur Aufrechterhaltung einer homogenen Katalysatorlösung eingesetzt werden. Zur Auflösung des Katalysators kann auch Methanol benutzt werden. Ein weiteres benutztes Lösungsmittel ist Xylol. Bei der Aufarbeitung muß dann der mehrwertige Alkohol azeotrop entfernt werden, was im Falle von Glykol beispielsweise mit p-Cymol gelingt, worauf sich nach Zusatz von Wasser eine azeotrope Entfernung des p-Cymols anschließt. Die genannte EP-Anmeldung berichtet von Arbeitstemperaturen von 100-200°C, Arbeitsdrücken von bis zu 16 bar und Ausbeuten im Bereich von 50-91 %.

Es wurde nun gefunden, daß man auf herkömmliche Alkoholate von einwertigen aliphatischen Alkoholen zurückgreifen kann, d.h., auf schwierig aufzuarbeitende Alkoholate des Glykols oder Glycerins verzichten kann, und dennoch unter Ausschluß von Kohlenwasserstoff-Lösungs mitteln, also entgegen der Lehre des Standes der Technik arbeiten kann.

Eine solche Verfahrensweise hat zunächst den Vorteil einer größeren Raumausbeute aufgrund der stärker konzentrierten Fahrweise. Des weiteren kann das Cyclopropancarbonsäureamid in kristalliner Form und damit in sehr hoher Reinheit gewonnen werden. Weiterhin ergeben sich sehr niedrige Arbeitsdrücke von höchstens 6 bar. Weitere Vorteile gehen aus der folgenden Beschreibung hervor.

Die Erfindung betrifft also ein Verfahren zur Herstellung von Cyclopropancarbonsäureamid durch Umsetzung von Cyclopropancarbonsäureester mit NH₃ in Gegenwart von katalytischen Mengen eines Alkoholats, das dadurch gekennzeichnet ist, daß ein Cyclopropancarbonsäureester der Formel 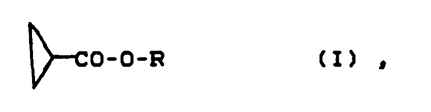
worin
- R: geradkettiges oder verzweigtes C₄-C₈-Alkyl darstellt,
in Gegenwart eines Alkalimetallalkoholats eines einwertigen C₁-C₈-Alkohols unter Ausschluß eines Kohlenvasserstoff-Lösungsmittels bei 60-200°C umgesetzt wird.

Der Esteralkohol des Cyclopropancarbonsäureesters hat 4-8 C-Atome und ist geradkettig oder verzweigt. In bevorzugter Weise hat dieser Alkohol 4-6 C-Atome, besonders bevorzugt 4 C-Atome; in ganz besonders bevorzugter Form wird der i-Butylester der Cyclopropancarbonsäure eingesetzt.

Das Alkalimetall des Alkoholats ist Lithium, Natrium, Kalium, Rubidium oder Cäsium, bevorzugt Natrium oder Kalium, besonders bevorzugt Natrium. Der Alkoholatalkohol hat 1-8, bevorzugt 1-6, besonders bevorzugt 1-4 C-Atome und ist geradkettig oder verzweigt. In ganz besonders bevorzugter Weise wird Natrium-i-butanolat eingesetzt. Eine weitere bevorzugte Ausführungsform benutzt als Alkoholatalkohol den gleichen, wie er im Ester vorliegt. Ganz besonders bevorzugt ist die Verwendung von i-Butanolat in Verbindung mit einem Cyclopropancarbonsäure-i-butylester. Das Alkoholat wird in einer Menge von 2-20 Mol-%, bevorzugt 4-16 Mol-%, besonders bevorzugt 6-14 Mol-%, bezogen auf die Molzahl des umzusetzenden Esters, eingesetzt.

Das erfindungsgemäße Verfahren wird unter Ausschluß eines Kohlenwasserstoff-Lösungsmittels durchgeführt. Das erfindungsgemäße Verfahren wird weiterhin so durchgeführt, daß zu Beginn der Reaktion ein Alkohol nur in dem Maße vorliegt, wie er zur Handhabung des Alkoholats, beispielsweise zum Dosieren in das Reaktionsgemisch, benötigt wird. Beispielsweise können viele Alkoholate, wie Natriummethylat, in fester Form ganz ohne alkoholische Hilfslösungsmittel gehandhabt werden. Bei höheren Alkoholaten ist es vielfach günstig, Schmelzen aus einem Gemisch mit ihrem zugrundeliegenden Alkohol zu dosieren; beispielsweise sei hier eine 35 gew.-%ige Schmelze von Natrium-i-butanolat in i-Butanol genannt. Für den Fall der Verwendung eines alkoholischen Hilfslösungsmittels für das Alkoholat sei allgemein eine 25-50 gew.-%ige Lösung oder Schmelze in einem Alkohol genannt, bevorzugt eine solche Lösung oder Schmelze in dem Alkohol, der dem Alkoholat zugrundeliegt. Das Vorhandensein von weiterem Alkohol im Verlaufe der Durchführung des erfindungsgemäßen Verfahrens kann außer Betracht bleiben, da bei jeder Ammonolyse von Estern der Esteralkohol abgespalten wird und in freier Form vorliegt.

Das erfindungsgemäße Verfahren wird bei 60-200°C, bevorzugt bei 70-160°C, besonders bevorzugt bei 80-140°C, durchgeführt. Durch den Ausschluß von Kohlenwasserstoff-Lösungsmitteln ist die Löslichkeit von Ammoniak im Reaktionsgemisch beträchtlich höher, so daß ein geringerer Druckanstieg verzeichnet wird. Dieser Effekt wird noch dadurch verstärkt, daß Ammoniak nachgesetzt wird, bevorzugt in dem Maße, wie die Reaktion fortschreitet. Das erfindungsgemäße Verfahren ist also dadurch weiterhin ausgezeichnet, daß bei einem Druck von höchstens 6 bar gearbeitet wird, wodurch in Apparaturen mit niedrigeren Sicherheitsanforderungen gearbeitet werden kann.

Zur Aufarbeitung eines Reaktionsansatzes wird dieser zunächst abgekühlt, wobei bereits während des Abkühlens kristallines Cyclopropancarbonsäureamid ausfällt. Die Kristallisation wird in der Nähe der Raumtemperatur, also bei 5-30°C, vervollständigt und eine Trennung von Kristallisat und Mutterlauge in bekannter Form, etwa durch Filtrieren, Zentrifugieren oder Dekantieren, bewirkt. Das Kristallisat kann durch kalten Alkohol, bevorzugt durch den Esteralkohol, gewaschen werden. Die Alkoholatmenge wird hierbei so bemessen, daß unter Berücksichtigung des abgespaltenen Esteralkohols die Sättigungskonzentration des Alkoholats im abgekühlten Reaktionsgemisch nicht überschritten wird. Bei einer solchen Arbeitsweise beträgt die erzielbare Ausbeute etwa 90 % bei einem über 99 %igen Umsatz. Neben dem analysenreinen auskristallisierten Cyclopropancarbonsäureamid wird eine Mutterlauge erhalten, die restliches Cyclopropancarbonsäureamid, abgespaltenen Esteralkohol und das Alkoholat enthält. Diese Mutterlauge kann eingeengt werden und nach Zusatz von Cyclopropancarbonsäureester erneut in die Ammonolyse zurückgeführt werden. Die Ausbeute ist dann nahezu quantitativ. Bei der Einengung der Mutterlauge zur Recyclisierung wird außer dem abzuziehenden überschüssigen Esteralkohol aus der vorangegangenen Durchführung des erfindungsgemäßen Verfahrens auch das gesamte Wasser, was etwa in diese alkoholathaltige Mutterlauge gelangt sein kann, als Azeotrop mit abgezogen. Damit kann eine Verunreinigung, etwa durch Verseifung des Esters oder Verseifung des Amids fast vollständig unterdrückt werden. Diese Aufarbeitungsvariante ist daher bevorzugt.

Neben der bevorzugten Aufarbeitungsvariante besteht aufgrund des nahezu vollständigen Umsatzes auch die Möglichkeit, auf die Rückführung des Alkoholats zu verzichten und durch Zusatz von Wasser und einer sauer reagierenden Substanz (beispielsweise Mineralsäuren oder sauer reagierende Ammoniumsalze) eine Hydrolyse und Neutralisation des Alkoholats durchzuführen und dann den abgespaltenen Esteralkohol als Azeotrop abzudestillieren. Bei dieser Fahrweise kann eine wäßrige Lösung des Cyclopropancarbonsäureamids erhalten werden, die beispielsweise direkt dem Hofmannschen Abbau zum Cyclopropylamin zugeführt werden kann.

Der Ausschluß von Kohlenwasserstoff-Lösungsmitteln bringt neben den obengenannten Vorteilen auch die Vermeidung zusätzlicher Azeotrope bei der Aufarbeitung und weiterhin die Vermeidung von Produktextraktionen, die mit aufzuarbeitenden Waschflüssigkeiten und Recyclisierungen verbunden sind.

### Beispiel 1

In einen 1,6-l-Autoklaven wurden 563,1 g Cyclopropancarbonsäure-i-butylester eingefüllt. Hierzu gab man 73,3 g 35 %ige Natrium-isobutanolat/iso-Butanol-Schmelze Die auf diese Weise erhaltene Mischung wurde nach zweimaliger Druckprobe mit N₂ auf 100°C aufgeheizt. Hierbei erhielt man eine leicht bräunliche Lösung. Nach Erreichen der Reaktionstemperatur drückte man 30 ml NH₃ (= 18,5 g) als NH₃ Gas auf, wodurch sich ein Druck von 6 bar einstellte, der recht schnell abfiel, so daß in der Folgezeit noch insgesamt 180 ml NH₃ (= 111 g) als Gas nachgedrückt wurden, bis der Druck auch nach 2 Stunden nicht mehr abfiel. Dann wurde noch in der Hitze vorsichtig entspannt und die Reaktionsmischung abgekühlt. Bei ca. 70°C fielen die ersten Kristalle aus ; der nach dem Kühlen auf 20°C erhaltene Kristallbrei wurde abgesaugt, mit 55 g i-BuOH gewaschen und getrocknet (50°C, 300 mbar).

### Ausbeute:

296 g Cyclopropancarbonsäureamid (Reinheit >99 % lt. GC) = 88 % der theoretischen Ausbeute und 331 g Mutterlauge.

Von der Mutterlauge wurden 200 g i-Butanol abgezogen, der Rest wurde in der nächsten Partie wieder eingesetzt; ein erneuter Zusatz von Natrium-isobutanolat war damit nicht nötig.

Ausbeute im 2. Ansatz: 332 g = 98 % der theoretischen Ausbeute.

### Beispiel 2

In einen 1,0-l-Autoklaven wurden 284,4 g Cyclopropancarbonsäure-i-butylester eingefüllt. Hierzu gab man 54,8 g 35 %ige Natrium-isobutanolat/iso-Butanol-Schmelze Die auf diese Weise erhaltene Mischung wurde nach zweimaliger Druckprobe mit N₂ auf 100°C aufgeheizt. Hierbei erhielt man eine leicht bräunliche Lösung. Nach Erreichen der Reaktionstemperatur drückte man 30 ml NH₃ (= 18,5 g) als Gas auf, wodurch sich ein Druck von 6 bar einstellte, der recht schnell abfiel, so daß in der Folgezeit noch insgesamt 38 ml NH₃ (= 23,5 g) als Gas nachgedrückt wurden, bis der Druck auch nach 2 Stunden nicht mehr abfiel (Gesamtreaktionsdauer: 5 bis 9 Stunden). Dann wurde noch in der Hitze vorsichtig entspannt und die Reaktionsmischung abgekühlt. Bei ca. 70°C fielen die ersten Kristalle aus. Zu dieser warmen, noch mit NH₃ gesättigten Reaktionsmischung setzte man nun 36 g einer 40 %igen wäßrigen (NH₄)₂SO₄-Lösung zu und destillierte über eine 20 cm-Vigreux-Kolonne NH₃, i-BuOH und H₂O ab, wobei durch ständigen Wasserzusatz gewährleistet wurde, daß nach dem weitgehenden Abdestillieren des Ammoniaks noch das i-BuOH/H₂O-Azeotrop überdestillierte und sich im Wasserabscheider eine Phasengrenze ausbildete.

Nach Beendigung der azeotropen i-BuOH-Entfernung destillierte man noch so lange Wasser ab, bis eine Sumpftemperatur von 140°C erreicht war (gesamte zugesetzte Wassermenge: 120 g; Gesamtmenge Destillat: 265 g an Wasser, i-BuOH und NH₃).

Nun ließ man erkalten, überführte den festen, kristallinen Reaktionsgefäßinhalt auf eine Glassinternutsche und saugte ab.

Ausbeute: 182,9 g 91,2 %iges feuchtes, salzhaltiges, kristallines Produkt = 98 % der theoretischen Ausbeute.

### Beispiel 3

Man verfuhr analog Beispiel 2, setzte jedoch statt der Ammoniumsulfatlösung 18 g 50 %ige Schwefelsäure zu. Die Aufarbeitung erfolgte wie im Beispiel 2. Ausbeute: 173,8 g 94,2 %iges feuchtes, salzhaltiges, kristallines Produkt = 96,2 % der theoretischen Ausbeute.

### Beispiel 4

Man verfuhr analog Beispiel 2, setzte jedoch statt Ammoniumsulfat 47 g 25 %ige Ammoniumchloridlösung zu und arbeitete die so erhaltene Reaktionsmischung ganz analog auf. Ausbeute: 192,0 g 85,9 %iges feuchtes, salzhaltiges, kristallines Produkt = 96,9 % der theoretischen Ausbeute.

### Beispiel 5

Man verfuhr analog Beispiel 2, ersetzte aber die i-Butanol/i-Butanolat-Menge durch 11 g Natriummethylat. Man arbeitete wie im Beispiel 2 auf. Ausbeute: 177,5 g 93,0 %iges feuchtes, salzhaltiges, kristallines Produkt = 97 % der theoretischen Ausbeute.

### Vergleichsbeispiel 1

96,4 Cyclopropancarbonsäure-isobutylester wurden mit 28,7 g 35 %iger Natrium-isobutylat/iso-Butanol-Schmelze und 285 ml i-Butanol versetzt. Man gab 18,6 g Ammoniak zu und erhitzte auf 80°C. Es stellte sich ein Druck von 4 bar ein. Nach 12 Stunden arbeitete man auf und erhielt 55,4 g Cyclopropancarbonsäureamid (96 % der theoretischen Ausbeute). Zur Durchführung war ein 1,0-l-Autoklav erforderlich. Das Reaktionsprodukt kristallisierte nicht aus, sondern konnte erst nach beträchtlichem Destillationsaufwand gewonnen werden.

### Vergleichsbeispiel 2

Man arbeitete wie im Vergleichsbeispiel 1, setzte jedoch statt i-Butanol 285 ml Xylol zu. Aufgrund der geringeren Löslichkeit von NH₃ stellte sich ein Druck von 12 bar ein. Zur Aufarbeitung wurde eine wäßrige (NH₄)₂SO₄-Lösung und soviel Wasser zugesetzt, bis 2 klare flüssige Phasen entstanden waren. Die wäßrige wurde eingeengt, bis Cyclopropancarbonsäureamid ausfiel und abfiltriert werden konnte. Ausbeute: 56,3 g Cyclopropancarbonsäureamid (97 % der Theorie).

## Patentansprüche

1. Verfahren zur Herstellung von Cyclopropancarbonsäureamid durch Umsetzung von Cyclopropancarbonsäureester mit Ammoniak in Gegenwart von katalytischen Mengen eines Alkoholats, dadurch gekennzeichnet, daß ein Cyclopropancarbonsäureester der Formel 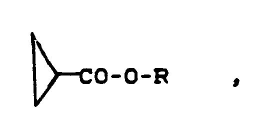 worin
R geradkettiges oder verzweigtes C₄-C₈-Alkyl darstellt,
in Gegenwart eines Alkalimetallalkoholats eines einwertigen C₁-C₈-Alkohols unter Ausschluß eines Kohlenwasserstoff-Lösungsmittels bei 60-200°C umgesetzt wird.

2. Verfahren nach Anspruch 1, dadurch gekennzeichnet, daß zu Beginn der Reaktion ein Alkohol nur in dem Maße vorliegt, wie er zur Handhabung des Alkoholats benötigt wird.

3. Verfahren nach Anspruch 2, dadurch gekennzeichnet, daß das Alkoholat in Form einer 25-50 gew.-%igen alkoholischen Lösung eingesetzt wird.

4. Verfahren nach Anspruch 1, dadurch gekennzeichnet, daß bei einem Druck von höchstens 6 bar gearbeitet wird.

5. Verfahren nach Anspruch 1, dadurch gekennzeichnet, daß der Esteralkoholrest R geradkettiges oder verzweigtes C₄-C₆-Alkyl, bevorzugt Butyl, besonders bevorzugt i-Butyl ist.

6. Verfahren nach Anspruch 1, dadurch gekennzeichnet, daß der Alkoholatalkohol 1-6 C-Atome, bevorzugt 1-4 C-Atome hat.

7. Verfahren nach Anspruch 6, dadurch gekennzeichnet, daß als Alkoholat Natrium-i-butanolat eingesetzt wird.

8. Verfahren nach Anspruch 1, dadurch gekennzeichnet, daß 2-20 Mol-% Alkoholat, bezogen auf die Molzahl umzusetzenden Esters, eingesetzt werden.

9. Verfahren nach Anspruch 1, dadurch gekennzeichnet, daß nach der Umsetzung das Cyclopropancarbonsäureamid als Kristallisat vom Reaktionsgemisch abgetrennt wird und hierbei die Sättigungskonzentration des Alkoholats im Reaktionsgemisch nicht überschritten wird.

10. Verfahren nach Anspruch 9, dadurch gekennzeichnet, daß das vom Kristallisat abgetrennte Reaktionsgemisch als Alkoholat recyclisiert wird.

## Claims

1. Process for the preparation of cyclopropanecarboxamide by reaction of cyclopropanecarboxylic ester with ammonia in the presence of catalytic amounts of an alcoholate, characterized in that a cyclopropanecarboxylic ester of the formula 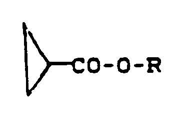 in which
R is straight-chain or branched C₄-C₈-alkyl
is reacted in the presence of an alkali metal alcoholate of a monohydric C₁-C₈-alcohol in the absence of a hydrocarbon solvent at 60-200°C.

2. Process according to Claim 1, characterized in that the amount of alcohol present at the beginning of the reaction is only such as is necessary for handling the alcoholate.

3. Process according to Claim 2, characterized in that the alcoholate is used in the form of a 25-50 % by weight alcoholic solution.

4. Process according to Claim 1, characterized in that the reaction is carried out at a maximum pressure of 6 bar.

5. Process according to Claim 1, characterized in that the ester alcohol radical R is straight-chain or branched C₄-C₆-alkyl, preferably butyl, particularly preferably isobutyl.

6. Process according to Claim 1, characterized in that the alcoholate alcohol has 1-6 C atoms, preferably 1-4 C atoms.

7. Process according to Claim 6, characterized in that the alcoholate used is sodium isobutoxide.

8. Process according to Claim 1, characterized in that 2-20 mol % of alcoholate, relative to the number of moles of the ester to be reacted, are used.

9. Process according to Claim 1, characterized in that after the reaction the cyclopropanecarboxamide is separated off from the reaction mixture in the form of crystals, without the saturation concentration of the alcoholate in the reaction mixture being exceeded.

10. Process according to Claim 9, characterized in that the reaction mixture which has been separated off from the crystals is recycled in the form of alcoholate.

## Revendications

1. Procédé de fabrication d'amide d'acide cyclopropane-carboxylique par mise en réaction d'ester d'acide cyclopropane-carboxylique avec de l'ammoniaque, en présence de quantités catalytiques d'un alcoolate, caractérisé en ce qu'un ester d'acide cyclopropane-carboxylique de la formule 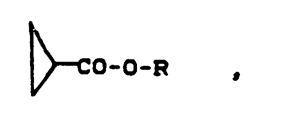 dans laquelle
R représente un alkyle de C₄ à C₈ à chaîne droite ou ramifiée,
est mis en réaction entre 60 et 200 °C, en présence d'un alcoolate de métal alcalin d'un alcool monovalent de C₁ à C₈ sous exclusion d'un solvant hydrocarboné.

2. Procédé selon la revendication 1, caractérisé en ce qu'au début de la réaction un alcool n'est présent que dans la mesure où il est nécessaire au maniement de l'alcoolate.

3. Procédé selon la revendication 2, caractérisé en ce que l'alcoolate est mis en oeuvre sous la forme d'une solution alcoolique à 20-50 % en poids.

4. Procédé selon la revendication 1, caractérisé en ce que l'on travaille à une pression de 6 bars au maximum.

5. Procédé selon la revendication 1, caractérisé en ce que le radical d'ester-alcool R est un alkyle de C₄ à C₆ à chaîne droite ou ramifiée, de préférence un butyle, avec une préférence particulière, un i-butyle.

6. Procédé selon la revendication 1, caractérisé en ce que l'alcool pour l'alcoolate possède 1 à 6, de préférence 1 à 4, atomes de C.

7. Procédé selon la revendication 6, caractérisé en ce que l'on utilise comme alcoolate du i-butanolate de sodium.

8. Procédé selon la revendication 1, caractérisé en ce que l'on met en oeuvre 2 à 20 moles % d'alcoolate par rapport au nombre de moles de l'ester à mettre en réaction.

9. Procédé selon la revendication 1, caractérisé en ce qu'après la mise en réaction, l'amide d'acide cyclopropanecarboxylique est séparé comme cristallisat du mélange réactionnel, la concentration de saturation de l'alcoolate dans le mélange réactionnel n'étant pas dépassée.

10. Procédé selon la revendication 9, caractérisé en ce que le mélange réactionnel séparé du cristallisat est recyclé comme alcoolate.
